# EUROPÄISCHE PATENTSCHRIFT

(11) **EP 2 222 261 B1**
(45) Veröffentlichungstag und Bekanntmachung des Hinweises auf die Patenterteilung: **09.05.2018**
(21) Anmeldenummer: 08848962.0
(22) Anmeldetag: 04.11.2008
(51) Int. Cl.: A61F 13/00, A61F 13/36, A61M 1/00

(54) **WUNDDRAINAGEAUFLAGE**
WOUND DRAINAGE COVERING
PANSEMENT DRAINANT

(30) Priorität: 13.11.2007 CH 17552007
(43) Veröffentlichungstag der Anmeldung: 01.09.2010
(73) Patentinhaber: Medela Holding AG, 6340 Baar (CH)
(72) Erfinder: LARSSON, Michael, 6300 Zug (CH)
(74) Vertreter: Clerc, Natalia
(86) Internationale Anmeldenummer: PCT/CH2008/000466
(87) Internationale Veröffentlichungsnummer: WO 2009/062327

(56) Entgegenhaltungen:
- WO-A-2006/048240
- WO-A-2007/030601
- DE-A1- 10 055 902
- DE-A1-102005 007 016
- US-A1- 2004 073 151
- US-A1- 2006 079 852

## Beschreibung

### Technisches Gebiet

Die Erfindung betrifft eine Wunddrainageauflage gemäss Oberbegriff des Patentanspruchs 1.

### Stand der Technik

Es ist bekannt, grosse oder schlecht heilende Wunden mit einer Unterdruck-Drainagevorrichtung zu behandeln. Die Wunde wird dabei mit einer Abdeckung, beispielsweise einer Folie oder einer steifen Haube, überdeckt, so dass ein Wundraum entsteht. In den Wundraum wird von aussen ein Drainageschlauch eingeführt, welcher mit einer Unterdruckpumpe verbunden ist, um Wundsekret aus der Wunde abzusaugen. Um den Wundraum zu füllen und insbesondere um den Unterdruck gleichmässig über die Wundoberfläche zu verteilen, wird eine Wunddrainageauflage auf die Wunde gelegt. Diese Wunddrainageauflage ist üblicherweise eine Schaumstoffeinlage mit entsprechend geeignet ausgebildeten Poren. Die Schaumstoffeinlage dient üblicherweise zudem als Absorptionskörper für das Wundsekret und muss entsprechend oft gewechselt werden. Entsprechende Wunddrainageauflagen sind beispielsweise aus WO 2006/056294, US 7 070 584, EP 1 284 777 und EP 0 620 720 bekannt. Eine Wunddrainageauflage mit einer Schaumstoffeinlage ausserhalb der luftdichten Deckschicht ist in WO 2006/052839 beschrieben.

Diese Schaumstoffeinlagen haben jedoch den Nachteil, dass sie bei Applikation eines Unterdrucks kollabieren oder ihre Poren zumindest verstopfen. Dadurch kann kein kontanter Fluidtransport aufrecht erhalten werden. Im schlimmsten Fall wird sogar der Transport des Wundsekrets blockiert.

WO 03/086232 offenbart ferner eine Wunddrainageauflage zur Auflage einer mittels Unterdruck zu behandelnden Wunde mit einer Lage, welche auf die Wundoberfläche gelegt wird und welche Durchgangslöcher und Kanäle zur Verteilung des Unterdrucks aufweist, und einer darüber angeordneten Deckschicht mit einer Zugangsöffnung für den Vakuumschlauch. Die Wundauflage ist steif und nicht komprimierbar ausgebildet. Sie ist ferner durchsichtig, so dass die Wundheilung beobachtet werden kann. Die Wunddrainageauflage kann mit Silberionen imprägniert sein.

In US 2002/0065494 wird eine ähnliche Wunddrainageauflage offenbart, wobei hier oberhalb der Deckschicht eine Gaze angeordnet ist, welche die Wundkavität bis zur Höhe der gesunden Haut hin auffüllt. Über dieser Gaze ist eine Folie angeordnet, welche Wasserdampf aus der Kavität entweichen lässt.

EP 0 099 758 zeigt eine Wundauflage zur Verwendung ohne Drainage, insbesondere zur Verwendung mit elektrischer Stimulation. Diese Wundauflage ist mehrschichtig aufgebaut, wobei sie eine halbdurchlässige Membran, eine durchlässige Verstärkungsschicht aus einem textilen Material und eine biologisch abbaubare, nicht haftende Berührungsschicht aufweist. Die Membran kontrolliert den Wasserdampftransfer weg von der Wunde. Auch in US 5 653 699 wird eine Wundauflage ohne Drainage beschrieben, welche zwecks Feuchthalten der Wunde den Wasserdampftransfer kontrollieren kann. Wundverbände mit Schichten, welche ein Fluid in eine nächsthöhere Schicht transportieren, sind beispielsweise auch in US 6 077 526 und WO 2004/060412 beschrieben.

US 5 989 478 offenbart ein Gewebe, welches als Decklage für Hygienebinden, Windeln oder Wundverbände vorgeschlagen wird. Das Gewebe ist flüssigkeitsdurchlässig und transportiert die Flüssigkeit aktiv von der einen Oberfläche zur gegenüberliegenden Oberfläche, wo sie an eine Sauglage abgegeben werden kann.

DE 10 2005 007 016 offenbart eine Wundauflage mit einem textilen, flüssigkeitsdurchlässigen Abstandsmaterial.

Ferner offenbaren DE 100 55 902, US2004073151 und WO 2007/030601 Auflagen, welche jeweils eine stark flüssigkeitsabsorbierende Lage aufweisen, um Flüssigkeit darin zu sammeln.

### Darstellung der Erfindung

Es ist eine Aufgabe der Erfindung, eine Wunddrainageauflage zu schaffen, welche einen optimalen Wundsekrettransport gewährleistet und trotzdem einfach aufgebaut ist. Diese Aufgabe löst eine Wunddrainageauflage mit den Merkmalen des Patentanspruchs 1.

Die erfindungsgemässe Wunddrainageauflage zur Auflage auf einer mittels Unterdruck zu behandelnden Wunde weist mindestens zwei übereinander angeordnete Lagen auf, wobei eine erste wundseitige Lage ein funktionales textiles Material ist und eine darüber angeordnete zweite Lage formstabil und flüssigkeitsdurchlässig ausgebildet ist.

Das funktionale textile Material ist mindestens in eine Richtung flüssigkeitsdurchlässig und ausserdem anisotrop ausgebildet.

Diese Wunddrainageauflage eignet sich zur Verwendung als Einlage in eine Wunde, welche mit einem Unterdruck beaufschlagt wird. Derartige Wunddrainagen eignen sich zur Heilung von Wunden von menschlichen und tierischen Patienten.

Die erste Lage und die zweite Lage können einschichtig bzw. über das gesamte Volumen gleich aufgebaut sein oder sie können jeweils aus einem Kompositmaterial bestehen. Die zweite Lage ist vorzugsweise grobmaschig oder mit anderweitig über ihre Fläche oder das gesamte Volumen verteilte grosse Öffnungen ausgebildet, so dass der applizierte Unterdruck gleichmässig verteilt werden kann und die Absaugkanäle der zweiten Lage nicht durch abgesaugtes Wundsekret verstopfen können.

Weitere vorteilhafte Ausführungsformen gehen aus den abhängigen Patentansprüchen hervor.

### Kurze Beschreibung der Zeichnungen

Im folgenden wird der Erfindungsgegenstand anhand eines bevorzugten Ausführungsbeispiels, welches in den beiliegenden Zeichnungen dargestellt ist, erläutert. Es zeigen:
- Figur 1: eine schematische Darstellung einer erfindungsgemässen Wunddrainageauflage und
- Figur 2: die Wunddrainageauflage gemäss Figur 1 in Verwendung in einer Wunde.

### Wege zur Ausführung der Erfindung

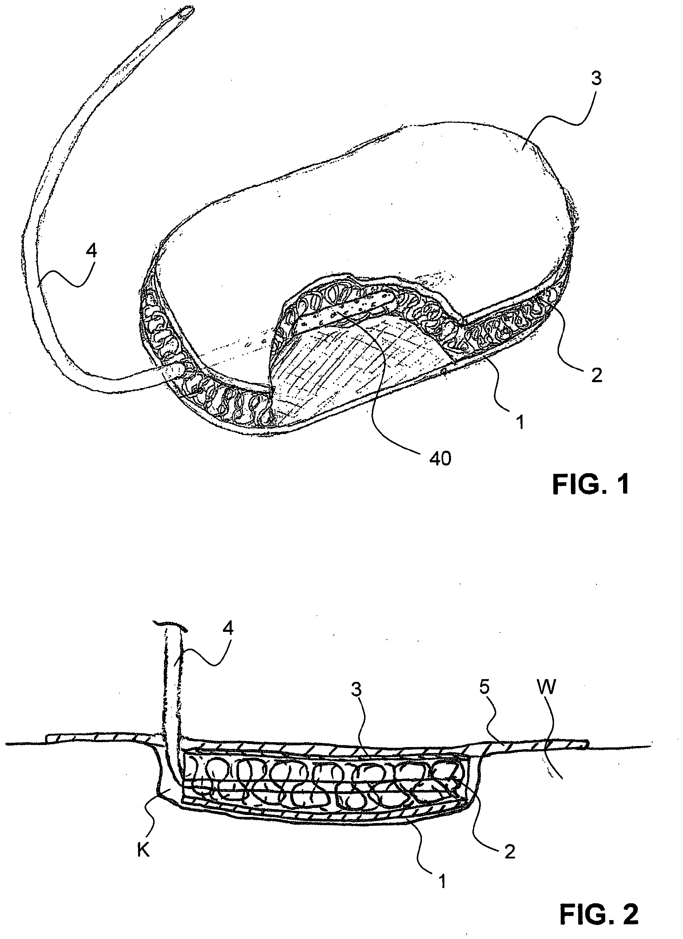

In Figur 1 ist ein bevorzugtes Ausführungsbeispiel der erfindungsgemässen Wunddrainageauflage bzw. des erfindungsgemässen Füllstoffes dargestellt. Sie dient, wie in Figur 2 erkennbar ist, zur Auflage auf eine Wunde W bzw. zur Einlage in eine Wundkavität K, wobei die Wunddrainageauflage mit einer luftdichten Abdeckung 5 vollständig überdeckt und somit die Wundkavität K luftdicht verschlossen ist. Die Abdeckung 5 kann eine starre Haube, eine flexible Folie oder ein anderes, aus dem Stand der Technik bekanntes Abdeckmittel sein. Vorzugsweise ist die Abdeckung 5 mindestens an ihren Rändern selbsthaftend, so dass sie auf die die Wunde umgebende gesunde Haut des Patienten luftdicht geklebt werden kann. Die Abdeckung 5 kann jedoch auch nicht selbsthaftend ausgeführt sein und mit zusätzlichen Befestigungsmitteln, insbesondere Klebstreifen, befestigt werden.

Von der Wunddrainageauflage führt ein Drainageschlauch 4 nach aussen. Dieser wird an eine Vakuum- oder Unterdruckpumpe angeschlossen, so dass in der Kavität K ein Unterdruck erzeugt werden kann und die in der Kavität K vorhandene Flüssigkeit, insbesondere das Wundsekret, abgesaugt werden kann. Typischerweise wird ein Unterdruck von 50 mmHg bis zu 220 mmHg erzeugt. Zusätzlich zum Drainageschlauch 4 können eine oder mehrere Zuleitungen durch die Abdeckung 5 in die Kavität K geführt sein. Durch diese Zuleitungen lassen sich Reinigungslösungen wie Natriumchlorid oder Medikamente wie beispielsweise Zinkoxid in die Wundkavität K einführen.

Wie in Figur 1 ersichtlich ist, ist die erfindungsgemässe Wunddrainageauflage schaumstofflos ausgebildet. Sie besteht aus mindestens zwei übereinander angeordneten Lagen oder Schichten, nämlich aus einer wundseitigen ersten Lage 1 aus einem funktionalen Material und einer darüber angeordneten zweiten Lage 2, welche formstabil bzw. steif ist und flüssigkeitsdurchlässig ausgebildet ist. Wie in Figur 1 dargestellt, kann zusätzlich noch eine dritte Lage 3 vorhanden sein, welche die zweite Lage 2 überdeckt und als flüssigkeitsdichte Deckschicht ausgebildet ist. Sie kann, muss aber nicht luftdicht sein.

Die erste Lage 1 ist aus einem funktionalen textilen Material gefertigt. Unter einem textilen Material wird hier ein Material verstanden, welches z.B. ein Gewebe oder Gewirke sein kann. Unter einem funktionalen Material wird hier ein Material verstanden, welches eine aktive Funktion, beispielsweise einen Flüssigkeitstransport oder eine Medikamentenabgabe, übernehmen kann. Als funktionales textiles Material eignen sich insbesondere die aus der Sportbekleidungsindustrie bekannten Materialien.

Vorzugsweise ist die erste Lage flexibel ausgebildet, insbesondere ist es ein flexibles Gewebe. Diese erste Lage 1 kann einschichtig ausgebildet sein. Vorzugsweise weist sie jedoch mehrere Schichten mit unterschiedlichen Funktionen auf. Vorzugsweise weist diese erste Lage 1 eine Dicke von 0.1 mm bis 5 mm auf. Vorzugsweise ist diese erste Lage 1 selbsttragend. Sie kann mit der zweiten Lage 2 verbunden sein, wobei sie in diesem Fall nicht zwingend selbsttragend sein muss.

Die Funktionalität des Materials kann verschiedenartig sein. Erfindungsgemäss ist die erste Lage 1 derart ausgebildet, dass sie einen aktiven bzw. selbsttätigen Flüssigkeitstransport durchführen kann. Das heisst, die Wundflüssigkeit wird auch ohne Applikation eines Unterdrucks von der Wunde weg in die nächst höher gelegene Lage, hier die zweite Lage 2, transportiert. Die erste Lage 1 weist hierfür Kapillaren auf. Derartige Materialien sind aus dem Stand der Technik bekannt, beispielsweise für Sportbekleidung oder wie eingangs erwähnt, für Hygienebinden, Windeln und Wundverbände ohne Drainage.

In einer weiteren bevorzugten Ausführungsform ist die erste Lage 1 zur zeitverzögerten und/oder wohldosierten Abgabe von Wirkstoffen ausgebildet. Sie kann beispielsweise Medikamente abgeben und/oder mit Silberionen beschichtet sein. Diese Funktionalität kann mit der Funktionalität des selbsttätigen Flüssigkeitstransports kombiniert sein. Andere Funktionalitäten, wie sie aus dem Gebiet der Nanotechnologie bekannt sind, sind ebenfalls möglich.

Die zweite Schicht oder Lage 2 ist relativ steif oder zumindest formstabil ausgebildet. Eine Adaption an Unebenheiten in der Wunde ist jedoch vorzugsweise möglich. Formstabil in diesem Zusammenhang soll bedeuten, dass diese zweite Lage 2 bei Applikation eines Unterdrucks nicht kollabiert und ihre inneren Hohlräume bzw. Kanäle nicht oder nur unwesentlich zusammengedrückt werden. Sie ist ferner flüssigkeitsdurchlässig und mit genügend grossen inneren Öffnungen ausgebildet, so dass das Wundsekret durch diese zweite Lage 2 abgesaugt werden kann, ohne dass diese zweite Lage 2 verstopft wird. Diese inneren Öffnungen sind vorzugsweise gleichmässig über die gesamte Fläche der zweiten Lage 2 verteilt, so dass der applizierte Unterdruck gleichmässig auf die Oberfläche der ersten Lage 1 und somit der Wunde verteilt werden kann. Vorzugsweise ist die zweite Lage 2 über das gesamte Volumen gleich ausgebildet, d.h. dass die inneren Öffnungen über das gesamte Volumen gleich verteilt sind. Es ist jedoch auch möglich, dass die Grösse, Anzahl und Verteilung der Öffnungen im wundnahen Bereich anders ist als im wundfemen Bereich der zweiten Lage 2. Vorzugsweise besteht diese zweite Lage aus einer einzigen Schicht. Sie kann aber auch mehrschichtig ausgebildet sein und die einzelnen Schichten können gleich oder verschieden voneinander ausgestaltet sein. So kann beispielsweise eine wundnahe Schicht der zweiten Lage 2 grössere bzw. mehr Öffnungen aufweisen als eine wundferne Schicht. Vorzugsweise ist die zweite Lage 2 ein grobmaschiges Gewebe, ein lockeres Gewirke oder ein Drahtgeflecht. Es kann z.B. aus einem Kunststoff oder aus einem Metall gefertigt sein. Die Dicke der zweiten Lage 2 ist abhängig von der Gestalt bzw. Tiefe der Wunde. Die zweite Lage 2 weist insbesondere eine um ein Vielfaches grössere Dicke auf als die erste Lage 1.

Die dritte Lage 3 ist flüssigkeitsdicht, vorzugsweise luftdicht ausgebildet. Sie ist beispielsweise ein einfaches Gewebe, eine Folie oder eine Beschichtung, welche auf der zweiten Lage 2 aufgebracht, insbesondere aufgeklebt oder aufgeschweisst ist. Vorzugsweise ist diese dritte Lage 3 durchsichtig ausgebildet, damit der Wundsekretstrom bzw. der Medikamentenstrom in der zweiten Lage 2 beobachtet werden kann. Auch die oben erwähnte Abdeckung 5 ist durchsichtig ausgebildet.
Der Drainageschlauch 4 ist in diesem Beispiel seitlich direkt, d.h. ohne Durchsetzung der dritten Lage 3, in die zweite Lage 2 eingeführt. Er kann jedoch auch die dritte Lage 3 durchsetzen. Innerhalb der zweiten Lage geht der Drainageschlauch 4 erfindungsgemäß in mehrere Verteilerrohre 40 über, welche über ihren Umfang verteilt angeordnete Absaugöffnungen aufweisen. Die Verteilerrohre 40 können am Schlauch 4 angeformt sein oder mit diesem zusammengesteckt werden. Diese Verteilerrohre 40 erstrecken sich annähernd über die gesamte Breite bzw. Länge der zweiten Lage. Die erfindungsgemässe Wunddrainageauflage ist einfach aufgebaut, weist dank der funktionalen ersten Lage eine gute Wirkungsweise auf und gewährleistet dank der formstabilen zweiten Lage einen optimalen Wundsekrettransport.

### Bezugszeichenliste

- 1: erste Lage
- 2: zweite Lage
- 3: dritte Lage
- 4: Drainageschlauch
- 40: Verteilerrohr
- 5: Abdeckung

## Patentansprüche

1. Wunddrainageauflage zur Auflage auf einer mittels Unterdruck zu behandelnden Wunde, wobei die Wunddrainageauflage geeignet ist, um von aussen einen Drainageschlauch in die Wunde einzuführen und diesen mit einer Unterdruckpumpe zu verbinden, um in der Wunde einen Unterdruck zu erzeugen und die in der Wunde vorhandene Wundflüssigkeit abzusaugen, wobei die Wunddrainageauflage mindestens zwei übereinander angeordnete Lagen aufweist, wobei eine erste wundseitige Lage (1) ein textiles Material ist und eine darüber angeordnete zweite Lage (2) formstabil und flüssigkeitsdurchlässig ausgebildet ist, **dadurch gekennzeichnet, dass** die erste Lage (1) aus einem anisotropen Material gefertigt ist, welches einen selbsttätigen Flüssigkeitstransport durchführen kann, so dass es Wundflüssigkeit ohne Applikation des Unterdrucks von der Wunde weg in die zweite Lage (2) transportiert, wobei die erste Lage (1) Kapillaren zum selbsttätigen Transport der Wundflüssigkeit in die zweite Lage (2) aufweist, dass über der zweiten Lage (2) eine dritte flüssigkeitsdichte Lage (3) angeordnet ist, wobei die dritte Lage (3) durchsichtig ist, so dass der Flüssigkeitsstrom in die zweite Lage (2) beobachtbar ist, und dass ein Drainageschlauch (4) in die zweite Lage (2) eingeführt ist, wobei der Drainageschlauch (4) in Verteilerrohre (40) übergeht, welche sich annähernd über die gesamte Breite bzw. Länge der zweiten Lage (2) erstrecken und welche über ihren Umfang verteilt angeordnete Absaugöffnungen aufweisen.

2. Wunddrainageauflage nach Anspruch 1, wobei die zweite Lage (2) ein Gewebe, ein Gewirke oder ein Drahtgeflecht ist.

3. Wunddrainageauflage nach einem der Ansprüche 1 oder 2, wobei das Material der ersten Lage (1) zur zeitverzögerten und/oder wohldosierten Abgabe von Wirkstoffen, insbesondere von Medikamenten und/oder Silberionen, geeignet ist.

4. Wunddrainageauflage nach einem der Ansprüche 1 bis 3, wobei das Material ein flexibles Gewebe ist.

5. Wunddrainageauflage nach einem der Ansprüche 1 bis 4, wobei die zweite Lage (2) eine vielfach grössere Dicke aufweist als die erste Lage (1).

6. Wunddrainageauflage nach einem der Ansprüche 1 bis 5, wobei sie schaumstofflos ist.

## Claims

1. A wound drainage dressing for placing on a wound that is to be treated by vacuum, wherein the wound drainage dressing is suitable for inserting a drainage hose into the wound from outside and for connecting this drainage hose to a vacuum pump in order to generate a vacuum in the wound and suck out the wound liquid present in the wound, wherein the wound drainage dressing has at least two layers arranged one on top of the other, wherein a first layer (1) facing the wound is a textile material, and a second layer (2), arranged over the first layer (1), is dimensionally stable and permeable to liquid, **characterized in that** the first layer (1) is made from an anisotropic material that is able to perform automatic transport of liquid, such that it transports wound liquid away from the wound into the second layer (2) without application of the vacuum, wherein the first layer (1) has capillaries for automatic transport of the wound liquid into the second layer (2), **in that** a third layer (3), impermeable to liquid, is arranged over the second layer (2), wherein the third layer (3) is transparent, such that the flow of liquid into the second layer (2) can be monitored, and **in that** a drainage hose (4) is inserted into the second layer (2), wherein the drainage hose (4) merges into distributor tubes (40), which extend approximately across the entire width or length of the second layer (2) and which have suction openings distributed about their circumference.

2. The wound drainage dressing as claimed in claim 1, wherein the second layer (2) is a woven fabric, a knitted fabric or a wire braid.

3. The wound drainage dressing as claimed in one of claims 1 or 2, wherein the material of the first layer (1) is suitable for delayed and/or precisely metered dispensing of active substances, in particular of medicaments and/or silver ions.

4. The wound drainage dressing as claimed in one of claims 1 through 3, wherein the material is a flexible woven fabric.

5. The wound drainage dressing as claimed in one of claims 1 through 4, wherein the second layer (2) has a thickness many times greater than the first layer (1).

6. The wound drainage dressing as claimed in one of claims 1 through 5, wherein said wound drainage dressing is foamless.

## Revendications

1. Un pansement drainant destiné à être appliqué sur une plaie à traiter par pression négative, où le pansement drainant est conçu pour permettre l'insertion d'un tuyau de drainage dans la plaie et le raccordement de celui-ci à une pompe à pression négative, pour générer un pression négative dans la plaie et aspirer le liquide de plaie présent dans la plaie, où le pansement drainant présente au moins deux couches superposées, où une première couche (1) côté plaie est un matériau textile et une seconde couche (2) disposée au-dessus de celle-ci est formée de façon perméable aux fluides et indéformable, **caractérisé en ce que** la première couche (1) est réalisée en matériau anisotropique, lequel peut effectuer un transport de liquide autonome, de sorte que celui-ci transporte du liquide de plaie sans application de pression négative depuis la plaie vers la seconde couche (2), où la première couche (1) présente des capillaires pour le transport autonome du liquide de plaie vers la seconde couche (2), qu'une troisième couche (3) imperméable aux fluides est disposée au-dessus de la seconde couche (2), où la troisième couche (3) est transparente, de sorte que l'écoulement de liquide dans la seconde couche (2) peut être observé, et qu'un tuyau de drainage est inséré dans la seconde couche (2), où le tuyau de drainage (2) passe à des tubes de distribution (40), lesquels s'étendent approximativement à travers la longueur respectivement la largeur intégrale de la seconde couche (2) et lesquels présentent des ouvertures d'aspiration disposées le long de leur circonférence.

2. Un pansement drainant selon la revendication 1, où la deuxième couche (2) est un tissu, un tricot ou un treillis.

3. Le pansement drainant selon une des revendications 1 ou 2, où le matériau de la première couche (1) est conçu pour une libération de principe actif, en particulier de médicaments et / ou d'ions d'argent, de manière retardée et / ou ajustée en dose.

4. Le pansement drainant selon une des revendications 1 à 3, où le matériau est un tissu flexible.

5. Le pansement drainant selon une des revendications 1 à 4, où la deuxième couche (2) présente une épaisseur plusieurs fois plus importante que la première couche (1).

6. Le pansement drainant selon une des revendications 1 à 5, où celui-ci est exempt de mousse.
